(19) Europäisches Patentamt
European Patent Office
Office européen des brevets

(11) **EP 4 700 069 A1**

(12) **EUROPEAN PATENT APPLICATION**
published in accordance with Art. 153(4) EPC

(43) Date of publication:
**25.02.2026 Bulletin 2026/09**

(51) International Patent Classification (IPC):
*C08G 81/00* (2006.01)   *A61F 13/532* (2006.01)
*A61F 13/535* (2006.01)   *B01J 20/26* (2006.01)
*B01J 20/30* (2006.01)   *C08F 8/50* (2006.01)

(21) Application number: **24831827.1**

(52) Cooperative Patent Classification (CPC):
**A61F 13/532; A61F 13/535; B01J 20/26;
B01J 20/30; C08F 8/50; C08G 81/00**

(22) Date of filing: **20.06.2024**

(86) International application number:
**PCT/JP2024/022447**

(87) International publication number:
**WO 2025/004966 (02.01.2025 Gazette 2025/01)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **30.06.2023 JP 2023107946**

(71) Applicant: **Sumitomo Seika Chemicals Co., Ltd.
Kako-gun, Hyogo 675-0145 (JP)**

(72) Inventor: **MAEDA, Hiroki
Himeji-shi, Hyogo 672-8076 (JP)**

(74) Representative: **Vossius & Partner
Patentanwälte Rechtsanwälte mbB
Siebertstraße 3
81675 München (DE)**

(54) **PRODUCTION METHOD FOR WATER-ABSORBING RESIN PARTICLES, ABSORBER, AND ABSORBENT ARTICLE**

(57)    A production method for water-absorbent resin particles containing a crosslinked polymer includes a preparation step of preparing a polymer solution that contains a polymer having a weight-average molecular weight of 300,000 or greater and a solvent, and a crosslinking step of crosslinking the polymer. The absorbent body contains water-absorbent resin particles obtained by the production method. The absorbent article contains the absorbent body.

**Fig.1**

EP 4 700 069 A1

## Description

### Technical Field

The present invention relates to a production method for water-absorbent resin particles, an absorbent body, and an absorbent article.

### Background Art

Generally, disposable sanitary products are configured to contain pulp fibers and a water-absorbent resin between a water-impermeable cover sheet and a water-permeable non-woven fabric, and the water-absorbent resin swells by containing moisture so that excrement can be absorbed. After such sanitary products are used, the sanitary products are treated by incineration or landfilling, but in recent years, the collection and reuse of components from used sanitary products have been examined. For example, Patent Literature 1 discloses a technique for decomposing a water-absorbent resin with a focus on cutting of a crosslinked portion in the water-absorbent resin, and a technique for collecting a water-soluble recycled polymer obtained by decomposing a water-absorbent resin as an aggregate by crosslinking with a multivalent metal ion.

### Citation List

### Patent Literature

[Patent Literature 1] Japanese Unexamined Patent Publication No. 2020-49398

## Summary of Invention

### Problem to be Solved

In many techniques disclosed as methods of regenerating a water-absorbent resin collected from used sanitary products, the water-absorbing ability of the water-absorbent resin is compared before and after a predetermined treatment, and in a case where the water-absorbing ability is more than or equal to the water-absorbing ability before the treatment, it is considered that the water-absorbent resin has been regenerated. However, since a crosslinked structure of the water-absorbent resin is destroyed by the predetermined treatment, the polymer component is likely to dissolve out, it is necessary to maintain the water-absorbing ability and to suppress an increase in the amount of dissolved polymer. However, in Patent Literature 1, it cannot be said that both the maintenance of the water-absorbing ability and the suppression of the dissolution of the polymer are achieved.

One aspect of the present invention relates to a method for producing water-absorbent resin particles having high water absorption performance and a small amount of dissolved matter, which is applicable to regenerating water-absorbent resin particles from a polymer formed by chemically decomposing a water-absorbent resin.

### Solution to Problem

One aspect of the present disclosure includes, for example, the following means.

[1] A production method for water-absorbent resin particles containing a crosslinked polymer, the production method including: a preparation step of preparing a polymer solution that contains a polymer having a weight-average molecular weight of 300,000 or greater and a solvent; and a crosslinking step of crosslinking the polymer.

[2] The production method according to [1], in which the preparation step includes a cleavage step of cleaving a crosslinked structure of the crosslinked polymer to obtain the polymer, and the cleavage step is performed in a treatment tank storing a reaction solution containing a water-absorbent resin and includes an operation of setting an oxygen concentration in a gas phase of the treatment tank to 18% by volume or less.

[3] The production method according to [1] or [2], in which the cleavage step includes an operation of increasing a temperature of the reaction solution to 50°C or higher.

[4] The production method according to any one of [1] to [3], in which the reaction solution contains at least one of an acid component or an alkaline component.

[5] The production method according to any one of [1] to [4], in which the crosslinking step includes crosslinking the polymer through a covalent bond.

[6] An absorbent body including: water-absorbent resin particles obtained by the production method according to any one of [1] to [5].

[7] An absorbent article including: the absorbent body according to [6].

**Advantageous Effects of Invention**

According to one aspect of the present invention, it is possible to provide a method for producing water-absorbent resin particles having high water absorption performance and a small amount of dissolved matter, which is applicable to the regeneration of water-absorbent resin particles from a polymer formed by chemically decomposing a water-absorbent resin.

**Brief Description of Drawings**

Fig. 1 is a cross-sectional view showing an example of an absorbent article having an absorbent body.
Fig. 2 is a schematic view showing a treatment tank that cleaves a crosslinked structure of a water-absorbent resin.

**Description of Embodiments**

The present invention is not limited to the following examples.

In the present specification, "(meth)acryl" means both acrylic and methacrylic. The terms "acrylate" and "methacrylate" also similarly denote "(meth)acrylate". The same also applies to other similar terms. The term "(poly)" includes both cases with and without the "poly" prefix. In the numerical value ranges described in a stepwise manner in the present specification, the upper limit or lower limit of a numerical value range in a certain step can be optionally combined with an upper limit or lower limit of a numerical value range in another step. In the numerical value ranges described in the present specification, an upper limit or lower limit of a numerical value range may be replaced with a value shown in examples. The materials described in the present specification may be used alone or in combination with two or more kinds thereof. "Physiological saline " denotes a 0.9 mass% sodium chloride aqueous solution. "Standard sieve" denotes a test sieve (metal mesh sieve) specified in JIS Z 8801-1:2019.

As an example of a production method for water-absorbent resin particles, the method includes a preparation step of preparing a polymer solution containing a polymer having a weight-average molecular weight of 300,000 or greater and a solvent, and a crosslinking step of crosslinking the polymer. In the method, the water-absorbent resin particles contain a crosslinked polymer. According to the method, for example, it is possible to regenerate water-absorbent resin particles from a polymer formed by chemically decomposing (specifically, cleaving a crosslinked structure of the water-absorbent resin) the water-absorbent resin and to produce water-absorbent resin particles having high water absorption performance and a small amount of dissolved matter. The water-absorbent resin to be subjected to chemical decomposition (hereinafter, also referred to as "water-absorbent resin for recycling") is preferably a used water-absorbent resin (that is, a water-absorbent resin in a gel state due to liquid absorption), but may be an unused water-absorbent resin (for example, a waste of the water-absorbent resin generated in a step of producing the water-absorbent resin), or may be a mixture of both the used and unused water-absorbent resins.

Examples of the absorbent article include diapers (for example, paper diapers), toilet training pants, incontinence pads, sanitary materials (sanitary napkins, tampons, and the like), sweat pads, pet sheets, portable toilet members, and animal excrement treatment materials. Used absorbent articles may also be employed. The water-absorbent resin for recycling, which is collected from the used water absorbent article, may form a gel by absorbing liquid during use.

The water-absorbent resin for recycling contains, for example, a polymer (crosslinked polymer) containing a structural unit derived from an ethylenically unsaturated monomer. Examples of the ethylenically unsaturated monomer include (meth)acrylic acid and a salt thereof, 2-(meth)acrylamide-2-methylpropanesulfonic acid and a salt thereof, (meth)acrylamide, N,N-dimethyl (meth)acrylamide, 2-hydroxyethyl (meth)acrylate, N-methylol (meth)acrylamide, polyethylene glycol mono(meth)acrylate, N,N-diethylaminoethyl (meth)acrylate, N,N-diethylaminopropyl (meth)acrylate, and diethylaminopropyl (meth)acrylamide. The ethylenically unsaturated monomer may include at least one compound selected from the group consisting of acrylic acid and a salt thereof, methacrylic acid and a salt thereof, acrylamide, methacrylamide, and N,N-dimethylacrylamide.

The crosslinked polymer may have a constitutional unit derived from a monomer other than an ethylenically unsaturated monomer. The proportion of the constitutional unit (particularly, (meth)acrylic acid and a salt thereof) derived from the ethylenically unsaturated monomer in the crosslinked polymer may be 70% to 100% by mole with respect to the total amount of the monomer unit. The proportion of the constitutional unit derived from (meth)acrylic acid and a salt thereof in the ethylenically unsaturated monomer may be 70% to 100% by mole.

The water-absorbent resin for recycling contains, for example, a crosslinked polymer having a poly(meth)acrylic acid structure. Examples of such a crosslinked polymer include a polymer obtained by polymerizing a monomer composition containing (meth)acrylic acid and a crosslinking agent capable of forming a covalent bond by reacting with a carboxyl group of (meth)acrylic acid, a polymer obtained by polymerizing a monomer composition containing (meth) acrylic acid to obtain a polymer and treating a surface of the polymer with a crosslinking agent capable of forming a covalent bond by reacting with a carboxyl group of the polymer, and a polymer obtained by polymerizing a monomer composition containing (meth)acrylic acid and a crosslinking agent capable of forming a covalent bond by reacting with a carboxyl group of (meth)acrylic acid to obtain a polymer and treating a surface of the polymer with a crosslinking agent capable of forming a covalent bond by reacting with a carboxyl group of the polymer.

The shape of the water-absorbent resin for recycling is not particularly limited, and the shape may be an amorphous lumpy shape in a case of a used gel and the shape may be an amorphous crushed shape, a scale-like shape, a granular shape, or the like in a case of an unused gel (typically, dry powder).

The preparation step can include a cleavage step of cleaving a crosslinked structure of a crosslinked polymer contained in the water-absorbent resin for recycling. The cleavage step is, for example, performed in a treatment tank storing a reaction solution that contains the water-absorbent resin for recycling and may include an operation of setting an oxygen concentration in a gas phase of the treatment tank to 18% by volume or less. In other words, the polymer in the preparation step can be obtained by cleaving the crosslinked structure of the crosslinked polymer while bringing a reaction solution that contains a water-absorbent resin for recycling (containing a crosslinked polymer), which is stored in a treatment tank, into contact with a gas phase in the treatment tank having an oxygen concentration of 18% by volume or less.

According to this method, the weight-average molecular weight of the polymer obtained by cleaving the crosslinked structure of the crosslinked polymer of the water-absorbent resin for recycling can be easily set to 300,000 or greater, and water-absorbent resin particles having high water absorption performance and a small amount of dissolved matter can be produced (regenerated) from the polymer.

From the viewpoint that the weight-average molecular weight of the polymer to be obtained is likely to be increased and the amount of the dissolved matter of the water-absorbent resin particles to be produced from the polymer is further decreased, the oxygen concentration in the gas phase of the treatment tank may be 16% by volume or less, 14% by volume or less, 12% by volume or less, 10% by volume or less, 8% by volume or less, 6% by volume or less, 4% by volume or less, 2% by volume or less, 1% by volume or less, 0.5% by volume or less, 0.1% by volume or less, or 0.05% by volume or less with respect to the total volume of the gas phase. The oxygen concentration in the gas phase may be substantially 0% by volume, greater than 0% by volume, or 0.01% by volume or greater. The oxygen concentration in the gas phase of the treatment tank may be 0% to 16% by volume, 0% to 12% by volume, 0% to 8% by volume, 0% to 6% by volume, 0% to 4% by volume, 0% to 1% by volume, 0% to 0.5% by volume, 0% to 0.1% by volume, 0% to 0.05% by volume, 0.01% to 0.5% by volume or less, 0.01% to 0.1% by volume or less, or 0.01% to 0.05% by volume or less. In order to set the oxygen concentration in the gas phase of the treatment tank to 18% by volume or less, for example, the gas phase of the treatment tank may be substituted with an inert gas (for example, nitrogen).

The reaction solution contains at least a water-absorbent resin containing a crosslinked polymer. The crosslinked polymer is dissolved in, for example, a solvent. The solvent of the reaction solution may be any solvent that can dissolve the crosslinked polymer, and examples thereof include water and an organic solvent.

The content of the water-absorbent resin in the reaction solution may be 30% by mass or less, 20% by mass or less, 15% by mass or less, 12% by mass or less, or 10% by mass or less, or may be 0.1% by mass or greater, 0.5% by mass or greater, 1% by mass or greater, or 1.5% by mass or greater with respect to the total amount of the reaction solution. The content of the crosslinked polymer in the reaction solution may be 0.1% to 30% by mass, 0.5% to 20% by mass, 1% to 15% by mass, or 1% to 10% by mass.

The reaction solution may contain components other than the water-absorbent resin. For example, the reaction solution may contain at least one of an acid component or an alkaline component (base component). From the viewpoint that the weight-average molecular weight of the polymer to be obtained is likely to be increased and the amount of the dissolved matter of the water-absorbent resin particles to be produced from the polymer is further decreased, the reaction solution may contain an alkaline component.

The acid component may be an inorganic acid, and may be, for example, at least one inorganic acid selected from the group consisting of acetic acid, nitric acid, sulfuric acid, hydrochloric acid, phosphoric acid, and boric acid, or at least one inorganic acid selected from sulfuric acid and hydrochloric acid.

The alkaline component (base component) may be an inorganic alkaline component (inorganic base component), and may be, for example, at least one inorganic alkaline component (inorganic base component) selected from the group consisting of sodium hydroxide, ammonia, potassium hydroxide, and calcium hydroxide, or at least one inorganic alkaline component (inorganic base component) selected from the group consisting of sodium hydroxide, potassium hydroxide, and calcium hydroxide.

The content of the acid component or the alkaline component (base component) in the reaction solution may be 0.003 mol/kg or greater, 0.01 mol/kg or greater, 0.1 mol/kg or greater, 0.2 mol/kg or greater, 0.4 mol/kg or greater, 0.5 mol/kg or greater, 0.6 mol/kg or greater, 0.7 mol/kg or greater, or 0.8 mol/kg or greater, or may be 2 mol/kg or less, 1.5 mol/kg or less, 1 mol/kg or less, 0.9 mol/kg or less, or 0.8 mol/kg or less with respect to the total amount of the reaction solution. The content of the acid component or the alkaline component (base component) in the reaction solution may be 0.003 to 2 mol/kg, 0.01 to 1.5 mol/kg, 0.1 to 1 mol/kg, 0.2 to 0.9 mol/kg, or 0.5 to 0.8 mol/kg with respect to the total amount of the reaction solution.

In a case where the reaction solution contains an acid component, the pH of the reaction solution may be 0.1 or greater, 0.2 or greater, 0.3 or greater, 0.5 or greater, 1.0 or greater, 1.5 or greater, or 2.0 or greater, and may be 5.0 or less, 4.5 or less, 4.0 or less, 3.5 or less, 3.0 or less, or 2.5 or less. In a case where the reaction solution contains an acid component, the pH of the reaction solution may be, for example, 0.1 to 5.0, 0.2 to 4.5, 0.3 to 4.0, 0.5 to 4.0, 1.0 to 3.5, 1.5 to 3.0, or 2.0 to 2.5.

In a case where the reaction solution contains an alkaline component (base component), the pH of the reaction solution may be 7.0 or greater, 7.5 or greater, 8.0 or greater, 8.5 or greater, 9.0 or greater, 9.5 or greater, or 10.0 or greater, and may be 14.0 or less, 13.5 or less, 13.0 or less, 12.5 or less, 12.0 or less, 11.5 or less, or 11.0 or less. In a case where the reaction solution contains an alkaline component (base component), the pH of the reaction solution may be, for example, 7.0 to 14.0, 7.5 to 13.5, 8.0 to 13.0, 8.5 to 12.5, 9.0 to 12.0, 9.5 to 11.5, or 10.0 to 11.0.

From the viewpoint that the weight-average molecular weight of the polymer to be obtained is likely to be increased and the amount of the dissolved matter of the water-absorbent resin particles to be produced from the polymer is further decreased, the cleavage step may include an operation of adjusting the temperature of the reaction solution. For example, the cleavage step may include an operation of adjusting the temperature of the reaction solution to 50°C or higher, 60°C or higher, 70°C or higher, 80°C or higher, or 90°C or higher, an operation of adjusting the temperature of the reaction solution to 150°C or lower, 120°C or lower, 110°C or lower, or 100°C or lower, or an operation of adjusting the temperature of the reaction solution to 50°C to 150°C, 60°C to 120°C, or 70°C to 100°C. The temperature of the reaction solution can be adjusted, for example, by heating or cooling the treatment tank.

The temperature of the reaction solution may be adjusted after the reaction solution and the gas phase in contact with the reaction solution reach an equilibrium state in the treatment tank. For example, the temperature of the reaction solution may be adjusted after the reaction solution and the gas phase are brought into contact with each other for 10 minutes or longer. The reaction solution may be stirred from the viewpoint of suppressing the deposition of the water-absorbent resin and the occurrence of temperature unevenness.

In a case where the water-absorbent resin for recycling contains a crosslinked polymer having a poly(meth)acrylic acid structure, the polymer obtained by cleaving the crosslinked polymer has a poly(meth)acrylic acid structure. In a case where the water-absorbent resin for recycling contains a crosslinked polymer having a poly(meth)acrylic acid structure, from the viewpoint of easily producing water-absorbent resin particles having high water absorption performance and a small amount of dissolved matter by avoiding cutting of the poly(meth)acrylic acid structure, the crosslinked polymer may be cleaved by bringing the water-absorbent resin for recycling into contact with an alkaline component (basic component).

From the viewpoint of further decreasing the amount of the dissolved matter of the water-absorbent resin particles to be produced, the weight-average molecular weight of the polymer (the polymer in the preparation step) obtained in the cleavage step may be 300,000 or greater, 400,000 or greater, 500,000 or greater, 700,000 or greater, 1,000,000 or greater, 1,300,000 or greater, 1,500,000 or greater, 1,700,000 or greater, 1,900,000 or greater, 2,000,000 or greater, 2,100,000 or greater, 2,200,000 or greater, 2,300,000 or greater, 2,400,000 or greater, or 2,500,000 or greater. The weight-average molecular weight of the polymer may be 10,000,000 or less, 8,000,000 or less, 6,000,000 or less, 4,000,000 or less, 3,500,000 or less, 3,000,000 or less, or 2,600,000 or less. The weight-average molecular weight of the polymer may be 300,000 to 10,000,000, 400,000 to 8,000,000, 500,000 to 6,000,000, 700,000 to 4,000,000, 1,000,000 to 3,500,000, 1,300,000 to 3,000,000, 1,500,000 to 2,600,000, 2,000,000 to 3,500,000, or 2,000,000 to 3,000,000. The weight-average molecular weight of the polymer can be measured by a method described in the examples below.

The solvent of the polymer solution may be any solvent that can dissolve the polymer, and examples thereof include water and organic solvents.

After a polymer is obtained by cleaving the crosslinked structure of the crosslinked polymer, solid-liquid separation may be carried out in order to remove impurities in the polymer solution before carrying out the crosslinking step. For example, the polymer solution may be filtered or ultrafiltered. Before the polymer solution is filtered, the pH of the polymer solution may be adjusted to near neutrality.

From the viewpoint of reducing the viscosity of the polymer solution, dilution, heating, addition of a salt (salt having a monovalent cation), or the like may be performed on the polymer solution after a polymer is obtained by cleaving the crosslinked structure of the crosslinked polymer and before the crosslinking step is performed.

The polymer solution may be subjected to a sterilization treatment after a polymer is obtained by cleaving the crosslinked structure of the crosslinked polymer, and before the crosslinking step is performed.

In the crosslinking step, for example, a solution containing a polymer and a crosslinking agent capable of forming a covalent bond with a functional group contained in the polymer is prepared, and the polymer is crosslinked through the covalent bond to obtain a crosslinked polymer. In the crosslinking step, the polymer solution may be gelled. In this case, the entire polymer solution loses fluidity, and a gel containing a crosslinked polymer and water is formed. In a case where the crosslinking proceeds to the extent that a gel is formed, it is particularly easy to obtain water-absorbent resin particles having high water absorption performance and a small amount of dissolved matter.

Examples of the functional group contained in the polymer include a carboxyl group. In a case where the polymer has a carboxyl group, the polymer is crosslinked by a reaction between the carboxyl group and a crosslinking agent and/or a reaction between the carboxyl groups. The covalent bond may be at least one selected from the group consisting of an ester bond, a thioester bond, an amide bond, an ether bond, and a carbon-carbon bond. The polymer may be crosslinked through at least one group selected from the group consisting of a carboxylic acid ester group, a thioester group, an amide group, an acid anhydride group, an oxyalkylene group, and an oxyarylene group.

Examples of the crosslinking agent include aliphatic polyhydric alcohols such as (poly)ethylene glycol, (poly) propylene glycol, (poly)glycerin, and pentaerythritol; glycidyl compounds such as (poly)ethylene glycol diglycidyl ether, (poly)propylene glycol diglycidyl ether, and (poly)glycerin diglycidyl ether; bisacrylamide compounds such as N,N'-methylene bis(meth)acrylamide; allylated starch; diallyl phthalate; N,N',N"-triallyl isocyanurate; divinylbenzene; ethylenediamine, polyethyleneimine, and glycidyl (meth)acrylate.

From the viewpoint of sufficiently crosslinking the polymer, the amount of the crosslinking agent may be 0.0001 parts by mass or more, 0.001 parts by mass or more, or 0.005 parts by mass or more, and may be 15 parts by mass or less, 10 parts by mass or less, or 5 parts by mass or less per 100 parts by mass of the polymer. The amount of the crosslinking agent may be 0.0001 parts by mass to 15 parts by mass, 0.001 parts by mass to 10 parts by mass, or 0.005 parts by mass to 5 parts by mass per 100 parts by mass of the polymer.

The reaction temperature in a case of crosslinking the polymer is appropriately set depending on the kind, the amount, and the like of the crosslinking agent to be used, but from the viewpoint of sufficiently crosslinking the polymer, the reaction temperature may be 50°C or higher, 80°C or higher, or 100°C or higher, and may be 220°C or lower, 200°C or lower, or 180°C or lower. The reaction temperature in a case of crosslinking the polymer may be 50°C to 220°C, 80°C to 200°C, or 100°C to 180°C.

The reaction time for crosslinking the polymer is appropriately set depending on the kind, the amount, the reaction temperature, and the like of the crosslinking agent to be used, but may be 1 minute to 200 minutes or 5 minutes to 150 minutes from the viewpoint of sufficiently crosslinking the polymer.

A surface crosslinking step of performing surface crosslinking of the crosslinked polymer may be carried out after the crosslinked polymer is obtained by performing the crosslinking step. The surface crosslinking can be performed, for example, by adding a crosslinking agent (surface crosslinking agent) for performing the surface crosslinking to the crosslinked polymer and carrying out the reaction.

The surface crosslinking agent may be a compound containing two or more reactive functional groups having reactivity with a functional group (for example, a carboxyl group) contained in the crosslinked polymer. The surface crosslinking agent may be the same as or different from the crosslinking agent in the crosslinking step.

The reactive functional group of the surface crosslinking agent may be a carbonate group, a alcoholic hydroxy group, an epoxy group, a halogeno group in a haloepoxy compound, an isocyanate group, an oxetanyl group, an oxazoline group, or a combination thereof. The carbonate group can react with the other two molecules, and thus is regarded as two reactive functional groups.

Examples of the surface crosslinking agent containing a carbonate group include alkylene carbonate (ethylene carbonate or the like). Examples of surface crosslinking agents containing an alcoholic hydroxyl group include polyol compounds such as ethylene glycol, propylene glycol, 1,4-butanediol, trimethylolpropane, glycerin, polyoxyethylene glycol, polyoxypropylene glycol, and polyglycerin, and hydroxyalkylamide compounds (bis[N,N-di($\beta$-hydroxyethyl)]adipamide and the like). Examples of the surface crosslinking agent containing two or more epoxy groups include (poly) ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether. Examples of haloepoxy compounds containing an epoxy group and a halogeno group include epichlorohydrin, epibromohydrin, and $\alpha$-methylepichlorohydrin. Examples of the surface crosslinking agent containing an isocyanate group include 2,4-tolylene diisocyanate and hexamethylene diisocyanate. Examples of surface crosslinking agents containing an oxetanyl group include 3-methyl-3-oxetane methanol, 3-ethyl-3-oxetane methanol, 3-butyl-3-oxetane methanol, 3-methyl-3-oxetane ethanol, 3-ethyl-3-oxetane ethanol, and 3-butyl-3-oxetane ethanol.

From the viewpoint of sufficiently achieving the surface crosslinking of the crosslinked polymer, the amount of the surface crosslinking agent may be 0.001 parts by mass or more, 0.005 parts by mass or more, or 0.01 parts by mass or more, and may be 5 parts by mass or less, 3 parts by mass or less, or 1 part by mass or less per 100 parts by mass of the crosslinked polymer. The amount of the crosslinking agent may be 0.001 parts by mass to 5 parts by mass, 0.005 parts by mass to 3 parts by mass, or 0.01 parts by mass to 1 part by mass per 100 parts by mass of the polymer.

A regenerated water-absorbent resin containing a crosslinked polymer is formed by a method including removing water from the crosslinked polymer obtained by performing an optional surface crosslinking step. In a case where the reaction solution to be provided in the crosslinking step is an aqueous solution, the water-absorbent resin particles are formed by a method including a drying step of removing water from a lumpy crosslinked polymer formed by gelation of the reaction solution itself to dry the crosslinked polymer so that a dried product is formed, and a pulverization step of pulverizing the dried product.

A method of drying the crosslinked polymer may be, for example, a typical method such as a squeezing method such as centrifugation, dehydration using an organic solvent, natural drying, heat drying, air drying, freeze drying, or a combination thereof. From the viewpoint of efficiently removing water, the heating temperature for drying may be 80°C to 220°C, 90°C to 200°C, or 100°C to 180°C.

The moisture content of the dried product may be, for example, 20% by mass or less, 10% by mass or less, or 5% by mass or less. The moisture content of the dried product is based on the total amount of the dried product. The moisture content denotes the proportion of moisture in the dried product. The moisture content of the dried product can be determined as the difference in mass of the crosslinked polymer before and after heating, when the crosslinked polymer containing water is heated at 200°C for 2 hours.

Before drying the crosslinked polymer, a coarsely crushed product having a structure with a small size to some extent may be formed by coarsely crushing the crosslinked polymer. Water can be efficiently removed by forming the coarsely crushed product. The structure constituting the coarsely crushed product can be, for example, an elongated structure, a granular structure (particle), or a combination thereof. The coarsely crushed product may have a plurality of structures having a shape that enables them to pass through a circular hole having a diameter of 10 mm or 7 mm. The elongated structure may be bent, and in a case where the maximum width thereof is 10 mm or less, it can be said that the elongated structure has a shape that enables the structure to pass through a circular hole having a diameter of 10 mm. The granular structure (particle) may be amorphous and may have a shape that enables the structure to pass through a circular hole having a diameter of 10 mm while changing the orientation. Examples of a coarse crusher for coarsely crushing the crosslinked polymer include a kneader (for example, a pressure type kneader or a double-arm type kneader), a meat chopper, a cutter mill, and a pharma mill.

The dried product is pulverized to form water-absorbent resin particles. The pulverization method is not particularly limited. For example, the dried product can be pulverized using a pulverizer such as a centrifugal pulverizer, a roller mill, a stamp mill, a jet mill, a high-speed rotary pulverizer, or a container drive type mill.

The water-absorbent resin particles obtained by pulverization may be classified. The classification denotes an operation of dividing a particle group (powder) into two or more particle groups having different particle size distributions. Some of the water-absorbent resin particles after the classification may be pulverized and classified again.

The classification method is not particularly limited, but may be, for example, screen classification or airflow classification. The screen classification is a method of classifying particles on a screen into particles that pass through meshes of the screen and particles that do not pass through the meshes by vibrating the screen. The screen classification can be performed by using, for example, a vibrating screen, a rotary sifter, a cylindrical stirring screen, a blower sifter, or a Ro-tap shaker. The airflow classification is a method of classifying particles using the flow of air.

The median particle diameter of the powder of the crosslinked polymer obtained by performing pulverization and, as necessary, classification may be, for example, 200 $\mu$m to 500 $\mu$m or 300 $\mu$m to 500 $\mu$m. The particle size distribution may be adjusted by mixing two or more powders having different median particle diameters, which are obtained by the classification.

From the viewpoint that the water-absorbent resin particles have high water absorption performance, the water retention amount of the water-absorbent resin particles with respect to the physiological saline may be 70 g/g or less, 68 g/g or less, 66 g/g or less, 64 g/g or less, 62 g/g or less, 60 g/g or less, or 58 g/g or less. The water retention amount of the water-absorbent resin particles with respect to the physiological saline may be 40 g/g or greater, 44 g/g or greater, 48 g/g or greater, 52 g/g or greater, or 56 g/g or greater. The water retention amount of the water-absorbent resin particles with respect to the physiological saline may be 40 to 70 g/g, 44 to 66 g/g, 48 to 62 g/g, 52 to 60 g/g, or 56 to 58 g/g. The water retention amount of the water-absorbent resin particles with respect to physiological saline is a value to be measured by a method described in the examples below.

The amount of the dissolved matter of the water-absorbent resin particles may be 0.45 g/g or less, 0.4 g/g or less, 0.35 g/g or less, 0.3 g/g or less, 0.28 g/g or less, 0.26 g/g or less, 0.24 g/g or less, 0.22 g/g or less, 0.2 g/g or less, or less than 0.2 g/g. The dissolution of the polymer can be suppressed as the amount of the dissolved matter of the water-absorbent resin particles decreases, and the dissolution of the polymer can be sufficiently suppressed in a case where the amount of the dissolved matter is 0.45 g/g or less. The lower limit of the amount of the dissolved matter of the water-absorbent resin particles is not particularly limited, but may be, for example, 0.01 g/g or greater, 0.02 g/g or greater, or 0.03 g/g or greater. The amount of the dissolved matter of the water-absorbent resin particles may be 0.01 to 0.45 g/g, 0.02 to 0.4 g/g, or 0.03 to 0.35 g/g. The amount of the dissolved matter of the water-absorbent resin particles is a value measured by a method described in examples below.

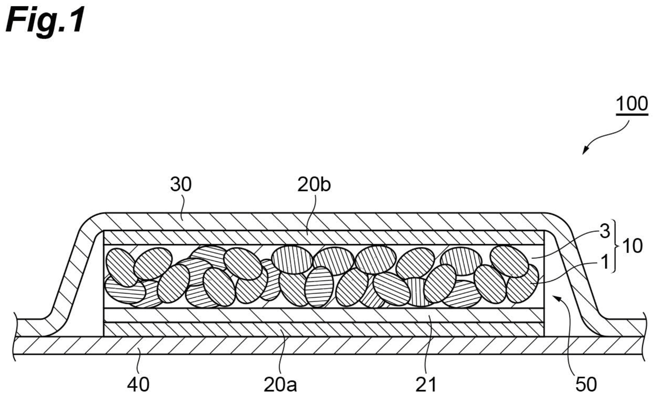

Fig. 1 is a cross-sectional view showing an example of an absorbent article having an absorbent body containing water-absorbent resin particles. An absorbent article 100 shown in Fig. 1 includes an water-absorbent sheet 50 having a film-like absorbent body 10, a liquid permeable sheet 30, and a liquid impermeable sheet 40.

The water-absorbent sheet 50 has an absorbent body 10 containing a powder of water-absorbent resin particles 1 and two sheets of core wrap sheets 20a and 20b. The absorbent body 10 is disposed inside the core wrap sheets 20a and 20b. The shape of the absorbent body 10 is retained by being sandwiched between the two core wrap sheets 20a and 20b. The core wrap sheets 20a and 20b may be two sheets, one turned-back sheet, or one bag body. A sheet member in which other constituent members are not provided on the outside of the core wrap sheets 20a and 20b that wrap the absorbent body 10 is also particularly referred to as a water-absorbent sheet.

The absorbent body 10 is a constituent member which mainly contains a powder of the water-absorbent resin particles 1 and in which the shape is retained to be constant. The absorbent body 10 may or may not include a fibrous substance 3 in addition to the powder of the water-absorbent resin particles 1. The content of the water-absorbent resin particles 1 in the absorbent body 10 may be 50% by mass or more and 100% by mass or less, 60% by mass or more and 100% by mass or less, 70% by mass or more and 100% by mass or less, 80% by mass or more and 100% by mass or less, or 90% by mass or more and 100% by mass or less with respect to the mass of the absorbent body 10.

The thickness of the absorbent body 10 may be, for example, 20 mm or less, 15 mm or less, 10 mm or less, 5 mm or less, 4 mm or less, or 3 mm or less, or may be 0.1 mm or more or 0.3 mm or more. The thickness of the absorbent body 10 may be 0.1 mm or more and 20 mm or less. The mass of the absorbent body 10 per unit area may be 1000 $g/m^2$ or less, 800 $g/m^2$ or less, or 600 $g/m^2$ or less, or may be 100 $g/m^2$ or greater.

The fibrous substance 3 can be, for example, cellulosic fibers, synthetic fibers, or a combination thereof. Examples of the cellulosic fibers include pulverized wood pulp, cotton, cotton linter, rayon, and cellulose acetate. Examples of the synthetic fibers include polyamide fibers, polyester fibers, and polyolefin fibers. The fibrous substance may be hydrophilic fiber (for example, pulp).

The absorbent body 10 may further contain an inorganic powder (for example, amorphous silica), a deodorant, an antibacterial agent, a fragrance, or the like. In a case where the water-absorbent resin particles 1 include inorganic particles, the absorbent body 10 may contain an inorganic powder in addition to the inorganic particles of the water-absorbent resin particles 1.

The water-absorbent sheet 50 may further contain an adhesive 21 sandwiched between the core wrap sheet 20a and the absorbent body 10. An adhesive layer may be sandwiched between the core wrap sheets 20a and 20b on both sides and the absorbent body 10. The adhesive 21 is not particularly limited, and may be, for example, a hot melt adhesive.

The core wrap sheets 20a and 20b may be, for example, non-woven fabrics. The two core wrap sheets 20a and 20b can be non-woven fabrics that are the same as or different from each other. The non-woven fabrics may be non-woven fabrics (short-fiber non-woven fabrics) formed of short fibers (that is, staple fibers), or may be non-woven fabrics (long-fiber non-woven fabrics) formed of long fibers (that is, filaments). The staples are not limited thereto, but may have a typical fiber length of several hundred mm or less.

The core wrap sheets 20a and 20b may be thermal bonded non-woven fabrics, air-through non-woven fabrics, resin bonded non-woven fabrics, spunbond non-woven fabrics, melt-blown non-woven fabrics, air-laid non-woven fabrics, spunlace non-woven fabrics, point-bonded non-woven fabrics, or a laminate containing two or more non-woven fabrics selected from these non-woven fabrics.

The non-woven fabric used as the core wrap sheets 20a and 20b can be non-woven fabrics formed of synthetic fibers, natural fibers, or a combination thereof. Examples of the synthetic fibers include fibers containing synthetic resins selected from polyolefin such as polyethylene (PE) and polypropylene (PP), polyester such as polyethylene terephthalate (PET), polytrimethylene terephthalate (PTT), and polyethylene naphthalate (PEN), polyamide such as nylon, and rayon. Examples of the natural fibers include fibers containing cotton, silk, hemp, or pulp (cellulose). Fibers forming the non-woven fabrics may be polyolefin fibers, polyester fibers, or a combination thereof. The core wrap sheets 20a and 20b may be tissue paper.

The water-absorbent sheet 50 may be used for producing various other absorbent articles. Examples of the absorbent articles include diapers (for example, paper diapers), toilet training pants, incontinence pads, sanitary materials (sanitary napkins, tampons, and the like), sweat pads, pet sheets, portable toilet members, and animal excrement treatment materials. The absorbent body constituting these absorbent articles frequently moves or is deformed due to the movement or the like of a user of the absorbent article.

The liquid permeable sheet 30 is disposed at the position of the outermost layer on a side where the liquid to be absorbed enters. The liquid permeable sheet 30 is disposed outside the core wrap sheet 20b in a state of being in contact with the core wrap sheet 20b. The liquid impermeable sheet 40 is disposed at the position of the outermost layer on the opposite side to the liquid permeable sheet 30 in the absorbent article 100. The liquid impermeable sheet 40 is disposed outside the core wrap sheet 20a in a state of being in contact with the core wrap sheet 20a. The liquid permeable sheet 30 and the liquid impermeable sheet 40 have a main surface wider than the main surface of the water-absorbent sheet 50, and outer edges of the liquid permeable sheet 30 and the liquid impermeable sheet 40 extend around the absorbent body 10 and the core wrap sheets 20a and 20b. However, the magnitude relationship between the absorbent body 10, the core wrap sheets 20a and 20b, the liquid permeable sheet 30, and the liquid impermeable sheet 40 is not particularly limited, and is appropriately adjusted depending on the applications of the absorbent article or the like.

The liquid permeable sheet 30 may be a non-woven fabric. The non-woven fabric used as the liquid permeable sheet 30 may have appropriate hydrophilicity from the viewpoint of the liquid absorption performance of the absorbent article. From this viewpoint, the liquid permeable sheet 30 may be a non-woven fabric having a hydrophilicity of 5 to 200, which is measured by a measuring method of the Japan Technical Association of Pulp and Paper Industry Test Method No. 68 (2000) of the Japan Technical Association of Pulp and Paper Industry. The hydrophilicity of the non-woven fabric may be 10 to 150. The details of Pulp and Paper Test Method No. 68 can be referred to, for example, WO2011/086843.

The non-woven fabric having hydrophilicity may be formed of fibers, such as rayon fibers exhibiting an appropriate hydrophilicity, or may be formed of fibers obtained by performing a hydrophilic treatment on hydrophobic chemical fibers such as polyolefin fibers and polyester fibers. Examples of a method of obtaining a non-woven fabric including hydrophobic chemical fibers that have been subjected to a hydrophilic treatment include a method of obtaining a non-woven fabric by a spunbond method using a mixture obtained by mixing a hydrophilic agent with hydrophobic chemical fibers, a method of accompanying a hydrophilic agent in preparing a spunbond non-woven fabric with hydrophobic chemical fibers, and a method of impregnating a spunbond non-woven fabric obtained using hydrophobic chemical fibers with a hydrophilic agent. As the hydrophilic agent, an anionic surfactant such as an aliphatic sulfonate or a higher alcohol sulfate ester salt, a cationic surfactant such as a quaternary ammonium salt, a nonionic surfactant such as polyethylene glycol fatty acid ester, polyglycerin fatty acid ester, or sorbitan fatty acid ester, a silicone surfactant such as polyoxyalkylene-modified silicone, a stain release agent formed of a polyester-based, polyamide-based, acrylic-based, or urethane-based resin, or the like is used.

From the viewpoint of imparting satisfactory liquid permeability, flexibility, strength, and cushioning properties to the absorbent article and from the viewpoint of accelerating the liquid permeation rate of the absorbent article, the basis weight (the mass per unit area) of the non-woven fabric used as the liquid permeable sheet 30 may be 5 g/m$^2$ to 200 g/m$^2$, 8 g/m$^2$ to 150 g/m$^2$, or 10 g/m$^2$ to 100 g/m$^2$. The thickness of the liquid permeable sheet 30 may be 20 $\mu$m to 1400 $\mu$m, 50 $\mu$m to 1200 $\mu$m, or 80 $\mu$m to 1000 $\mu$m.

The liquid impermeable sheet 40 prevents a liquid absorbed by the absorbent body 10 from leaking to the outside from the liquid impermeable sheet 40 side. The liquid impermeable sheet 40 may be a resin sheet or a non-woven fabric. The resin sheet may be a sheet formed of a synthetic resin such as polyethylene, polypropylene, and polyvinyl chloride. The non-woven fabric may be a spunbond/melt-blown/spunbond (SMS) non-woven fabric in which a waterproof melt-blown non-woven fabric is sandwiched between high-strength spunbond non-woven fabrics. The liquid impermeable sheet 40 may be a composite sheet of a resin sheet and a non-woven fabric (for example, spunbond non-woven fabric, spunlace non-woven fabric). The liquid impermeable sheet 40 may have breathability from the viewpoint that dampness generated at the time of wearing is reduced, and thereby discomfort to a wearer can be reduced. As the liquid impermeable sheet 40 having breathability, it is possible to use, for example, a sheet of low-density polyethylene (LDPE) resin.

From the viewpoint of ensuring the flexibility so that the feeling of wearing the absorbent article is not impaired, the basis weight (mass per unit area) of the liquid impermeable sheet 40 may be 10 g/m$^2$ to 50 g/m$^2$.

**Examples**

Hereinafter, the present invention will be described in more detail with reference to the Examples.

<Evaluation method>

The weight-average molecular weight Mw, the water retention amount, and the dissolved matter were measured by the following evaluation methods. The measurement results are as shown in Tables 1 and 2.

[Weight-average molecular weight Mw]

Measuring method: light scattering GPC
Autosampler: Autosampler AS-11 (manufactured by FLOM CO., LTD.)
Degassing device: Gastorr AG-16 (manufactured by FLOM CO., LTD.)
Liquid feeding unit: LC-10AD (manufactured by Shimadzu Corporation)
Column: OHpak SB-807HQ, SB-806HQ, SB-804HQ (Shodex Series, manufactured by Resonac Corporation)
Detector: Triple Detector TDA 302 (manufactured by Viscotec).
Eluent: NaNO$_3$ (0.2 mol/L)/methylparaben (2 mmol/L)/distilled water
Measurement conditions: injection volume of 500 $\mu$L, flow rate of 0.5 mL/min, column/detector temperature of 40°C, dn/dC 0.2270

A sample solution adjusted to contain 0.01 g of the polymer was placed in a 300 mL beaker, the above-described eluent was added thereto such that the total amount reached 100 mL, and the solution was stirred at 250 rpm for 1 hour. In a case where the pH of the sample solution was not neutral, the pH was adjusted to 7 using 1 mol/L hydrochloric acid (NAKALAI TESQUE, Inc.) or a 1 mol/L sodium hydroxide aqueous solution (NAKALAI TESQUE, Inc.). The weight-average molecular weight Mw is measured by GPC using a filtrate obtained by passing the sample solution after being stirred through a filter syringe having a pore size of 0.8 μm.

[Water Retention Amount]

The water retention amount (room temperature, 25°C ± 2°C) of the regenerated water-absorbent resin particles in physiological saline was measured by the following procedures. First, a cotton bag (cotton broadcloth No. 60, 100 mm in width × 200 mm in length) in which 2.0 g of the regenerated water-absorbent resin particles were weighed was placed in a beaker having an internal volume of 500 mL. After 500 g of physiological saline was poured into the cotton bag containing the regenerated water-absorbent resin particles at once so that lumps could not be produced, the upper part of the cotton bag was bound with a rubber band, and the cotton bag was allowed to stand for 30 minutes to swell the regenerated water-absorbent resin particles. The cotton bag after 30 minutes was dehydrated for 1 minute using a dehydrator (manufactured by KOKUSAN Co., Ltd., product number: H-122) which had been set to have a centrifugal force of 167 G, and the mass Wa [g] of the cotton bag containing the swollen gel after dehydration was measured. The same operation was performed without adding the regenerated water-absorbent resin particles, the empty mass Wb [g] in a case where the cotton bag was wet was measured, and the water retention amount of the regenerated water-absorbent resin particles in the physiological saline was calculated from the following equation.

$$\text{Water retention amount } [g/g] = (Wa - Wb)/2.0$$

[Dissolved matter]

The amount of the dissolved matter of the regenerated water-absorbent resin was measured in an environment of a temperature of 25°C ± 2°C and a humidity of 50% ± 10%. 500 g of physiological saline in a 500 mL beaker was stirred with a stirrer (cylindrical shape having a diameter of 8 mm and a length of 30 mm, no ring) rotating at 600 rpm. The temperature of the physiological saline was 25°C. 2.000 g of the regenerated water-absorbent resin particles were added thereto, and the dispersion liquid containing the regenerated water-absorbent resin particles was stirred for 3 hours. The dispersion liquid was filtered through a standard sieve having an opening of 75 μm, and the filtrate was collected. 80 g of the obtained filtrate was weighed in a 100 mL beaker which had been weighed in advance to a constant weight at 140°C. The filtrate in the beaker was heated in a blast dryer (FV-320, manufactured by ADVANTEC) at 140°C for 15 hours to remove the moisture, and the mass Wc (g) of the remaining solid component was measured. A blank test was performed by the same operation as the operation described above without putting the regenerated water-absorbent resin particles into physiological saline, and the mass Wd (g) of the solid component remaining in the beaker was measured. The amount of the dissolved matter was calculated according to the following equation.

$$\text{Dissolved matter } [g/g] = ((Wc - Wd)/80) \times 500/2.000$$

(Example 1)

A commercially available paper diaper (manufactured by Kao Corporation, Merries Pants Smooth Air Through, L size) was prepared, and water-absorbent resin containing a crosslinked polymer containing acrylic acid and an acrylic acid salt as monomer units were collected from the paper diaper.

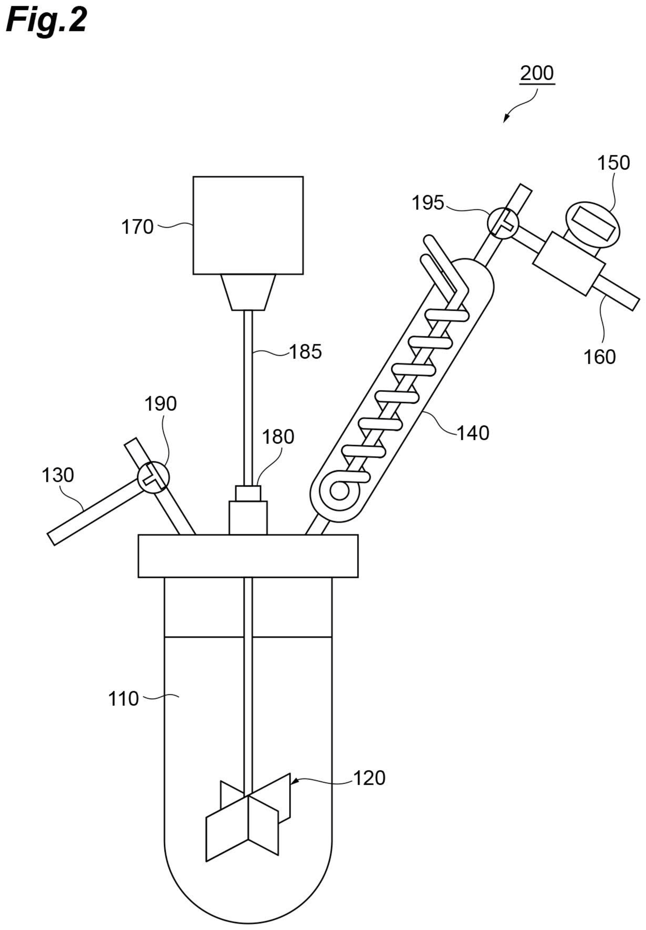

As shown in Fig. 2, a reflux cooling device, a nitrogen gas introduction pipe, and a stirrer (a 150 mL round-bottomed cylindrical separable flask equipped with a stirrer blade having four inclined paddle blades with a blade diameter of 40 mm, manufactured by EYELA) were prepared. 1 g of the water-absorbent resin collected from the paper diaper was weighed in a separable flask, 50 g of ion-exchanged water was added thereto, and the solution was allowed to stand for 5 minutes to swell the water-absorbent resin. Next, 50 g of 1.6 mol/kg sulfuric acid was added to the separable flask, and the stirring of the solution was started at 300 rpm. Nitrogen gas having an oxygen concentration of 0.02% by volume was blown into the separable flask at 200 mL/min for 15 minutes, and the inside of the separable flask was substituted with nitrogen. Thereafter, an increase in temperature was started using a personal organic synthesis reactor (manufactured by EYELA), and the crosslinked structure of the crosslinked polymer of the water-absorbent resin was cleaved while the internal temperature was maintained at 100°C for 24 hours. A decomposition solution (aqueous solution of water-soluble recycled polymer, pH of 0.3) containing a polymer which was a water-absorbent resin solubilized by cleavage was obtained. In a case where the weight-average molecular weight Mw of the obtained polymer was measured by light scattering GPC, the weight-average molecular weight Mw thereof was 2,292,000.

(Example 2)

A decomposition solution (aqueous solution of water-soluble recycled polymer) containing a polymer which was a water-absorbent resin solubilized by cleavage was obtained by performing the same operation as in Example 1 except that 50 g of 1.6 mol/kg hydrochloric acid was used instead of 50 g of 1.6 mol/kg sulfuric acid. In a case where the weight-average molecular weight Mw of the obtained polymer was measured by light scattering GPC, the weight-average molecular weight Mw thereof was 2,378,000.

(Example 3)

A decomposition solution (aqueous solution of water-soluble recycled polymer, pH of 13.3) containing a polymer which was a water-absorbent resin solubilized by cleavage was obtained by performing the same operation as in Example 1 except that 50 g of a 1.6 mol/kg sodium hydroxide aqueous solution was used instead of 50 g of a 1.6 mol/kg sulfuric acid. In a case where the weight-average molecular weight Mw of the obtained polymer was measured by light scattering GPC, the weight-average molecular weight Mw thereof was 2,594,000.

(Example 4)

A reflux cooling device, a nitrogen gas introduction pipe, and a stirrer (a 4 L round-bottomed cylindrical separable flask equipped with a stirrer blade having four inclined paddle blades with a blade diameter of 50 mm in two stages, manufactured by DURAN) were prepared. 90 g of the water-absorbent resin collected from the paper diaper was weighed in the separable flask, 1,710 g of ion-exchanged water was added thereto, and the solution was allowed to stand for 5 minutes to swell the water-absorbent resin. Next, 1,200 g of a 1.5 mol/kg sodium hydroxide aqueous solution was added to the separable flask, and stirring of the solution at 160 rpm was started. Nitrogen gas was blown into the separable flask at 200 mL/min for 15 minutes to substitute the inside of the separable flask with nitrogen. Thereafter, the crosslinked structure of the crosslinked polymer of the water-absorbent resin was cleaved while the internal temperature was maintained at 80°C for 24 hours. A decomposition solution (aqueous solution of water-soluble recycled polymer, pH of 13.1) containing a polymer which was a water-absorbent resin solubilized by cleavage was obtained. In a case where the weight-average molecular weight Mw of the obtained polymer was measured by light scattering GPC, the weight-average molecular weight Mw thereof was 2,490,000.

(Example 5)

100 g of an aqueous solution of the water-soluble recycled polymer obtained in Example 4 was weighed in a 500 mL polybeaker, and 200 g of ion-exchanged water was added thereto. Stirring of the solution at 300 rpm was started using a stirrer equipped with a stirrer blade having four inclined paddle blades with a blade diameter of 50 mm in two stages. 25 g of a cation exchange resin (DOWEX 50W $\times$ 8, strong acid ion exchange resin (H type), manufactured by DuPont) was added thereto, and the solution was stirred for 30 minutes and filtered through a nylon mesh having an opening of 25 $\mu$m to remove the cation exchange resin. The pH of the aqueous solution of the obtained water-soluble recycled polymer was 6.8.

A reflux cooling device, a nitrogen gas introduction pipe, and a stirrer (150 mL round-bottomed cylindrical separable flask equipped with a stirrer blade having four inclined paddle blades with a blade diameter of 40 mm, manufactured by EYELA) were prepared. 100 g of an aqueous solution of the obtained water-soluble recycled polymer (weight-average molecular weight Mw of 2,490,000, pH of 6.8) was weighed in a separable flask and stirred at 300 rpm. Nitrogen gas was blown into the separable flask at 200 mL/min for 15 minutes to substitute the inside of the separable flask with nitrogen. Thereafter, an increase in temperature was started using a personal organic synthesis reactor (manufactured by EYELA), and the internal temperature was maintained at 70°C for 24 hours. In a case where the weight-average molecular weight Mw of the polymer in the aqueous solution of the water-soluble recycled polymer after heating was measured by light scattering GPC, the weight-average molecular weight Mw thereof was 2,530,000.

(Example 6)

A decomposition solution (aqueous solution of water-soluble recycled polymer) containing a polymer which was a water-absorbent resin solubilized by cleavage was obtained by performing the same operation as in Example 1 except that air having an oxygen concentration of 3.1% by volume was blown instead of the nitrogen gas in a case where the inside of the separable flask was substituted with nitrogen. In a case where the weight-average molecular weight Mw of the obtained polymer was measured by light scattering GPC, the weight-average molecular weight Mw was 1,939,000.

(Example 7)

A decomposition solution (aqueous solution of water-soluble recycled polymer, pH of 0.2) containing a polymer which was a water-absorbent resin solubilized by cleavage was obtained by performing the same operation as in Example 1 except that air having an oxygen concentration of 5.3% by volume was blown instead of the nitrogen gas in a case where the inside of the separable flask was substituted with nitrogen. In a case where the weight-average molecular weight Mw of the obtained polymer was measured by light scattering GPC, the weight-average molecular weight Mw was 1,153,000.

(Example 8)

A decomposition solution (aqueous solution of water-soluble recycled polymer, pH of 0.2) containing a polymer which was a water-absorbent resin solubilized by cleavage was obtained by performing the same operation as in Example 1 except that air having an oxygen concentration of 10.3% by volume was blown instead of the nitrogen gas in a case where the inside of the separable flask was substituted with nitrogen. In a case where the weight-average molecular weight Mw of the obtained polymer was measured by light scattering GPC, the weight-average molecular weight Mw was 1,036,000.

(Example 9)

A decomposition solution (aqueous solution of water-soluble recycled polymer, pH of 0.3) containing a polymer which was a water-absorbent resin solubilized by cleavage was obtained by performing the same operation as in Example 1 except that air having an oxygen concentration of 15.1% by volume was blown instead of the nitrogen gas in a case where the inside of the separable flask was substituted with nitrogen. In a case where the weight-average molecular weight Mw of the obtained polymer was measured by light scattering GPC, the weight-average molecular weight Mw was 860,510.

(Example 10)

A decomposition solution (aqueous solution of water-soluble recycled polymer, pH of 0.2) containing a polymer which was a water-absorbent resin solubilized by cleavage was obtained by performing the same operation as in Example 1 except that air having an oxygen concentration of 17.3% by volume was blown instead of the nitrogen gas in a case where the inside of the separable flask was substituted with nitrogen. In a case where the weight-average molecular weight Mw of the obtained polymer was measured by light scattering GPC, the weight-average molecular weight Mw was 538,236.

(Example 11)

A decomposition solution (aqueous solution of water-soluble recycled polymer) containing a polymer which was a water-absorbent resin solubilized by cleavage was obtained by performing the same operation as in Example 1 except that air having an oxygen concentration of 20.1 % by volume was blown instead of the nitrogen gas in a case where the inside of the separable flask was substituted with nitrogen. In a case where the weight-average molecular weight Mw of the obtained polymer was measured by light scattering GPC, the weight-average molecular weight Mw was 480,296.

(Comparative Example 1)

A decomposition solution (aqueous solution of water-soluble recycled polymer) containing a polymer which was a water-absorbent resin solubilized by cleavage was obtained by performing the same operation as in Example 2 except that air having an oxygen concentration of 20.1 % by volume was blown instead of the nitrogen gas in a case where the inside of the separable flask was substituted with nitrogen. In a case where the weight-average molecular weight Mw of the obtained polymer was measured by light scattering GPC, the weight-average molecular weight Mw was 144,006.

(Comparative Example 2)

A decomposition solution (aqueous solution of water-soluble recycled polymer) containing a polymer which was a water-absorbent resin solubilized by cleavage was obtained by performing the same operation as in Example 5 except that air having an oxygen concentration of 20.1 % by volume was blown instead of the nitrogen gas in a case where the inside of the separable flask was substituted with nitrogen. In a case where the weight-average molecular weight Mw of the obtained polymer was measured by light scattering GPC, the weight-average molecular weight Mw was 285,786.

(Example 12)

20.0 g of a commercially available polyacrylic acid partially neutralized product (AQUPAANA AP-70, manufactured by Sumitomo Seika Chemicals Co., Ltd.) was weighed in a 500 mL polybeaker, and dissolved in 180.0 g of ion-exchanged water to prepare a 10 mass% aqueous solution. In a case where the weight-average molecular weight Mw was measured by light scattering GPC, the weight-average molecular weight Mw thereof was 3,136,000. 1.00 g of a 2 mass% ethylene glycol diglycidyl ether aqueous solution was added to the aqueous solution, and the solution was stirred at 650 rpm for 5 minutes using a hand mixer (MK-H4, manufactured by Panasonic Corporation). The aqueous solution after being stirred was transferred to a stainless steel vat ($135 \times 170$ mm) coated with PTFE and dried at 115°C for 1 hour with a hot air dryer (FV-320, manufactured by ADVANTEC Co., Ltd.) to obtain a gel. The obtained gel was divided into a size of about 3 cm and dried at 115°C for 2 hours using a hot air dryer. Thereafter, the dried product was pulverized using a centrifugal pulverizer (ZM200, manufactured by Verder Scientific, screen diameter of 1 mm, 6,000 rpm). The pulverized product was classified using a sieve, and regenerated water-absorbent resin particles having a particle diameter of 180 to 850 $\mu$m were collected. In a case where the water retention amount and the amount of the dissolved matter of the regenerated water-absorbent resin particles were measured, the water retention amount thereof was 57.2 g/g and the amount of the dissolved matter was 0.199 g/g.

(Example 13)

20.0 g of a commercially available polyacrylic acid partially neutralized product (AQUPAANA AP-70, manufactured by Sumitomo Seika Chemicals Co., Ltd.) was weighed in a 500 mL polybeaker, and dissolved in 180.0 g of ion-exchanged water to prepare a 10 mass% aqueous solution. This solution was uniformly spread on a stainless steel vat ($250 \times 185$ mm) and irradiated with ultraviolet rays for 60 minutes at an irradiation distance of 5 cm using a UV lamp (Spectro-UV Fluorescent Bench & Display Lamps: xx-15NF, wavelength: 254 nm), the weight-average molecular weight Mw was measured by light scattering GPC, and as a result, the weight-average molecular weight Mw thereof was 535,228. This aqueous solution was returned to a 500 mL polybeaker, 4.48 g of a 2 mass% ethylene glycol diglycidyl ether aqueous solution was added thereto, and the solution was stirred at 650 rpm for 5 minutes using a hand mixer (MK-H4, manufactured by Panasonic Corporation). The aqueous solution after being stirred was transferred to a stainless steel vat ($135 \times 170$ mm) coated with PTFE and dried at 115°C for 1 hour with a hot air dryer (FV-320, manufactured by ADVANTEC Co., Ltd.) to obtain a gel. The obtained gel was divided into a size of about 3 cm and dried at 115°C for 2 hours using a hot air dryer. Thereafter, the dried product was pulverized using a centrifugal pulverizer (ZM200, manufactured by Verder Scientific, screen diameter of 1 mm, 6,000 rpm). The pulverized product was classified using a sieve, and regenerated water-absorbent resin particles having a particle diameter of 180 to 850 $\mu$m were collected. In a case where the water retention amount and the amount of the dissolved matter of the regenerated water-absorbent resin particles were measured, the water retention amount was 57.9 g/g and the amount of the dissolved matter was 0.251 g/g.

(Comparative Example 3)

20.0 g of a commercially available polyacrylic acid partially neutralized product (AQUPAANA AP-70, manufactured by Sumitomo Seika Chemicals Co., Ltd.) was weighed in a 500 mL polybeaker, and dissolved in 180.0 g of ion-exchanged water to prepare a 10 mass% aqueous solution. In order to reduce the molecular weight, this solution was uniformly spread on a stainless steel vat ($250 \times 185$ mm) and irradiated with ultraviolet rays for 230 hours at an irradiation distance of 5 cm using a UV lamp (Spectro-UV Fluorescent Bench & Display Lamps: xx-15NF, wavelength: 254 nm), the weight-average molecular weight Mw was measured by light scattering GPC, and as a result, the weight-average molecular weight Mw thereof was 260,968. This aqueous solution was returned to a 500 mL polybeaker, 8.40 g of a 2 mass% ethylene glycol diglycidyl ether aqueous solution was added thereto, and the solution was stirred at 650 rpm for 5 minutes using a hand mixer (MK-H4, manufactured by Panasonic Corporation). The aqueous solution after being stirred was transferred to a stainless steel vat ($135 \times 170$ mm) coated with PTFE and dried at 115°C for 1 hour with a hot air dryer (FV-320, manufactured by ADVANTEC Co., Ltd.) to obtain a gel. The obtained gel was divided into a size of about 3 cm and dried at 115°C for 2 hours using a hot air dryer. Thereafter, the dried product was pulverized using a centrifugal pulverizer (ZM200, manufactured by Verder Scientific, screen diameter of 1 mm, 6,000 rpm). The pulverized product was classified using a sieve, and regenerated water-absorbent resin particles having a particle diameter of 180 to 850 $\mu$m were rcollected. In a case where the water retention amount and the amount of the dissolved matter of the regenerated water-absorbent resin particles were measured, the water retention amount was 78.9 g/g and the amount of the dissolved matter was 0.492 g/g.

[Table 1]

| | Conditions for step of cleaving crosslinked structure of crosslinked polymer | | | | | Weight-average molecular weight Mw |
|---|---|---|---|---|---|---|
| | Concentration of water-absorbent resin [% by mass] | Acid and base components | Concentration of acid and base components [mol/kg] | Reaction temperature (internal temperature) [°C] | Oxygen concentration [% by volume] | |
| Example 1 | 1.0 | Sulfuric acid | 0.8 | 100 | 0.02 | 2292000 |
| Example 2 | 1.0 | Hydrochloric acid | 0.8 | 100 | 0.02 | 2378000 |
| Example 3 | 1.0 | Sodium hydroxide | 0.8 | 100 | 0.02 | 2594000 |
| Example 4 | 3.0 | Sodium hydroxide | 0.6 | 80 | 0.02 | 2490000 |
| Example 5 | 1.0 | - | - | 70 | 0.02 | 2530000 |
| Example 6 | 1.0 | Sulfuric acid | 0.8 | 100 | 3.1 | 1939000 |
| Example 7 | 1.0 | Sulfuric acid | 0.8 | 100 | 5.3 | 1153000 |
| Example 8 | 1.0 | Sulfuric acid | 0.8 | 100 | 10.3 | 1036000 |
| Example 9 | 1.0 | Sulfuric acid | 0.8 | 100 | 15.1 | 860510 |
| Example 10 | 1.0 | Sulfuric acid | 0.8 | 100 | 17.3 | 538236 |
| Example 11 | 1.0 | Sulfuric acid | 0.8 | 100 | 20.1 | 480296 |
| Comparative Example 1 | 1.0 | Hydrochloric acid | 0.8 | 100 | 20.1 | 144006 |
| Comparative Example 2 | 1.0 | - | - | 70 | 20.1 | 285786 |

[Table 2]

| | Weight-average molecular weight Mw | Water retention amount [g/g] | Dissolved matter [g/g] |
|---|---|---|---|
| Example 12 | 3136000 | 57.2 | 0.199 |
| Example 13 | 535228 | 57.9 | 0.251 |
| Comparative Example 3 | 260968 | 78.9 | 0.492 |

As shown in the results of Examples 12 and 13 and Comparative Example 3, it was found that the regenerated water-absorbent resin particles obtained by using a polymer having a weight-average molecular weight of 300,000 or greater were water-absorbent resin particles with high water absorption performance and a small amount of dissolved matter due to an appropriately high water retention amount and a small amount of the dissolved matter.

Further, as shown in the results of Examples 1 to 10 and Comparative Examples 1 and 2, it was found that a polymer having a large weight-average molecular weight was obtained by decreasing the oxygen concentration in the step of cleaving the crosslinked structure of the crosslinked polymer and, particularly, it was found that a polymer having a weight-average molecular weight of 500,000 or greater was obtained by setting the oxygen concentration to 18% by volume or less.

**Reference Signs List**

1: water-absorbent resin particle
10: absorbent body

20a, 20b: core wrap sheet
30: liquid permeable sheet
40: liquid impermeable sheet
50: water-absorbent sheet
100: absorbent article
110: round-bottomed cylindrical separable flask
120: four inclined paddle blades
130: nitrogen gas introduction pipe
140: reflux cooling device
150: oxygen concentration meter
160: gas discharge pipe
170: stirring motor
180: shaft holder
185: stirring shaft
190, 195: three-way cock
200: treatment tank

**Claims**

1. A production method for water-absorbent resin particles comprising a crosslinked polymer, the production method comprising:

   a preparation step of preparing a polymer solution that comprises a polymer having a weight-average molecular weight of 300,000 or greater and a solvent; and
   a crosslinking step of crosslinking the polymer.

2. The production method according to Claim 1,

   wherein the preparation step comprises a cleavage step of cleaving a crosslinked structure of the crosslinked polymer to obtain the polymer, and
   the cleavage step is performed in a treatment tank storing a reaction solution comprising a water-absorbent resin and comprises an operation of setting an oxygen concentration in a gas phase of the treatment tank to 18% by volume or less.

3. The production method according to Claim 2,
   wherein the cleavage step comprises an operation of increasing a temperature of the reaction solution to 50°C or higher.

4. The production method according to Claim 2 or 3,
   wherein the reaction solution comprises at least one of an acid component or an alkaline component.

5. The production method according to any one of Claims 1 to 3,
   wherein the crosslinking step comprises crosslinking the polymer through a covalent bond.

6. An absorbent body comprising:
   water-absorbent resin particles obtained by the production method according to any one of Claims 1 to 3.

7. An absorbent article comprising:
   the absorbent body according to Claim 6.

Fig.1

# Fig.2

## INTERNATIONAL SEARCH REPORT

| International application No. |
| --- |
| **PCT/JP2024/022447** |

### A. CLASSIFICATION OF SUBJECT MATTER

*C08G 81/00*(2006.01)i; *A61F 13/532*(2006.01)i; *A61F 13/535*(2006.01)i; *B01J 20/26*(2006.01)i; *B01J 20/30*(2006.01)i; *C08F 8/50*(2006.01)i

FI: C08G81/00; A61F13/532 100; A61F13/535 100; B01J20/26 D; B01J20/30; C08F8/50

According to International Patent Classification (IPC) or to both national classification and IPC

### B. FIELDS SEARCHED

Minimum documentation searched (classification system followed by classification symbols)

C08G81/00; A61F13/532; A61F13/535; B01J20/26; B01J20/30; C08F8/50

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| X | WO 2016/158976 A1 (NIPPON SHOKUBAI CO., LTD.) 06 October 2016 (2016-10-06) claims, paragraphs [0047]-[0049], [0083]-[0090], [0147] | 1, 5-7 |
| A | | 2-4 |
| A | JP 2012-219172 A (MITSUI CHEMICALS, INC.) 12 November 2012 (2012-11-12) | 1-7 |
| A | WO 2020/213298 A1 (SANYO CHEMICAL INDUSTRIES, LTD.) 22 October 2020 (2020-10-22) | 1-7 |
| A | WO 2023/120597 A1 (UNI-CHARM CO., LTD.) 29 June 2023 (2023-06-29) | 1-7 |

☐ Further documents are listed in the continuation of Box C.  ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **30 July 2024** | **13 August 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

## INTERNATIONAL SEARCH REPORT
### Information on patent family members

International application No.

**PCT/JP2024/022447**

| Patent document cited in search report | | | Publication date (day/month/year) | Patent family member(s) | | | Publication date (day/month/year) |
|---|---|---|---|---|---|---|---|
| WO | 2016/158976 | A1 | 06 October 2016 | US | 2018/0094131 | A1 | |
| | | | | claims, paragraphs [0089]-[0092], [0131]-[0137], [0200] | | | |
| | | | | EP | 3278873 | A1 | |
| | | | | CN | 107405600 | A | |
| | | | | KR | 10-2017-0132800 | A | |
| JP | 2012-219172 | A | 12 November 2012 | (Family: none) | | | |
| WO | 2020/213298 | A1 | 22 October 2020 | US | 2022/0212165 | A1 | |
| | | | | EP | 3957679 | A1 | |
| | | | | CN | 113710730 | A | |
| WO | 2023/120597 | A1 | 29 June 2023 | (Family: none) | | | |

Form PCT/ISA/210 (patent family annex) (July 2022)

19

**REFERENCES CITED IN THE DESCRIPTION**

**Patent documents cited in the description**

- JP 2020049398 A

- WO 2011086843 A